# EUROPÄISCHE PATENTSCHRIFT

(11) **EP 1 725 288 B1**
(45) Veröffentlichungstag und Bekanntmachung des Hinweises auf die Patenterteilung: **01.01.2014**
(21) Anmeldenummer: 05735779.0
(22) Anmeldetag: 01.03.2005
(51) Int. Cl.: A61M 15/00

(54) **PULVERINHALATOR MIT MEHRKANALDÜSE**
POWDER INHALER COMPRISING A MULTI-CHANNEL NOZZLE
INHALATEUR DE POUDRE COMPRENANT UNE BUSE A CANAUX MULTIPLES

(30) Priorität: 05.03.2004 DE 102004012093
(43) Veröffentlichungstag der Anmeldung: 29.11.2006
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: HOCHRAINER, Dieter, 57392 Schmallenberg (DE); DUNNE, Stephen Terence, Stowmarket, Suffolk IP14 3AE (GB); BOECK, Georg, 88471 Laupheim (DE); SCHIEWE, Joerg, 55129 Mainz (DE)
(74) Vertreter: Simon, Elke Anna Maria
(86) Internationale Anmeldenummer: PCT/EP2005/002131
(87) Internationale Veröffentlichungsnummer: WO 2005/084735

(56) Entgegenhaltungen:
- EP-A- 1 062 962
- WO-A-01/41846
- US-A- 5 683 361
- US-A1- 2002 033 177

## Beschreibung

Die Erfindung betrifft einen Pulverinhalator mit Mundstück zur Dispergierung von pharmazeutischen Arzneistoff-Formulierungen, welcher über eine Hilfsenergiequelle in Form eines Druckmediumsystems verfügt, mit einer Vorrichtung zur Bevorratung einer Pulver-Formulierung, wobei bei Aktivierung des Druckmediumsystems ein von dem Druckmediumsystem freigesetztes, gasförmiges Druckmedium mit der Pulver-Formulierung ein Aerosol in der Art ausbildet, dass die Pulverpartikel in dem gasförmigen Druckmedium dispergiert vorliegen.

Derartige Pulverinhalatoren werden für die Bereitstellung inhalierfähiger Arzneistoffe benötigt. Insbesondere bei Erkrankungen im Lungen- und Bronchialbereich werden die Arzneistoffe als inhalierfähige Arzneistoffe (Inhalativa) benötigt und zur Verfügung gestellt.

Im Rahmen der vorliegenden Erfindung ist unter dem Begriff "Arzneistoff` der wirksame Bestandteil eines Arzneimittels zu verstehen, der üblicherweise auch als Pharmakon oder Wirkstoff bezeichnet wird.

Der Begriff "Arzneistoff-Formulierung" bezieht sich auf pulverförmige Formulierungen, die den Arzneistoff beinhalten und zur inhalativen Applikation am Mensch oder Tier geeignet sind. Üblicherweise wird der Wirkstoff mit Adjuvantien und Additiven vermischt.

Üblicherweise werden in Inhalatoren die Formulierungen in einem Reservoir gelagert bzw. bevorratet, weshalb es notwendig ist, dass die verwendeten Formulierungen eine ausreichende Lagerstabilität aufweisen. Hierzu können dem Arzneistoff Hilfsstoffe zugesetzt werden, mit denen die physiko-chemischen Eigenschaften eingestellt werden, die die qualitätsbestimmenden Parameter wie Verfügbarkeit und Haltbarkeit in gewünschter Weise beeinflussen.

Die in dem Pulverinhalator bevorratete Arzneistoff-Formulierung wird zerstäubt und als Aerosol vom Patienten eingeatmet. Dabei wird der Arzneistoff in inhalierfähiger Form bereitgestellt.

Normalerweise wird jedoch bei diesem Vorgang nicht die gesamte **abgemessene Dosis** als Aerosol ausgebracht, sondern nur ein Teil davon. Das liegt daran, dass ein Teil der Pulverformulierung im Vorratsgefäß zurückbleibt, nur aufgewirbelt wird und sich dann an einer anderen Stelle des Inhalators wieder abscheidet.

Der Teil der abgemessenen Dosis, der das Mundstück des Pulverinhalators verlässt, wird als **ausgebrachte Dosis** bezeichnet. Pulverteilchen können beim Inhaliervorgang nur dann in die Lunge gelangen, wenn der aerodynamische Partikeldurchmesser kleiner als 5 µm ist. Die Konsequenz ist, dass nur ein Teil der ausgebrachten Dosis auch tatsächlich in die Lunge gelangen kann. Dieser Anteil kann nur durch aufwendige Versuche am Patienten bestimmt werden. Aus diesem Grund wurden in-vitro Tests entwickelt, mit denen in einem einfachen Laborexperiment der **aerodynamische Feinanteil** ermittelt wird, der mit dem lungengängigen Teil der ausgebrachten Dosis korreliert. Der aerodynamische Feinanteil ist definiert als der Anteil der abgemessenen Dosis in Prozent, der einen aerodynamischen Partikeldurchmesser kleiner als 5,8 µm besitzt.

Im Rahmen der vorliegenden Erfindung ist unter dem aerodynamischen Partikeldurchmesser der Teilchendurchmesser zu verstehen, der dem Äquivalentdurchmesser einer Kugel der Dichte von 1g/cm³ entspricht, die die gleiche Sedimentationsgeschwindigkeit in Luft besitzt wie das untersuchte Teilchen.

Um einen möglichst hohen aerodynamischen Feinpartikelanteil zu erzielen, sind folgende Überlegungen zielführend.

Zunächst muss eine Pulverformulierung bereitgestellt werden, die den Arzneistoff in mikronisiertem Form enthält. Der überwiegende Teil aller Arzneistoffpartikel sollte eine Größe im Bereich von 1 - 5 µm besitzen. Da mikronisierte Pulver als Haufwerke eine hohe Tendenz zur Ausbildung von Partikelagglomeraten zeigen, enthält die Pulverformulierung meist Hilfsstoffe, die die Deagglomeration der mikronisierten Arzneistoffpartikel erleichtern und auch die Fließfähigkeit erhöhen. Ein weiterer qualitätsrelevanter Parameter für die Pulverformulierung ist deren chemische und physikalische Stabilität. Chemische Stabilität ist gewährleistet, wenn der Arzneistoff sich bei der Lagerung nicht in Zersetzungsprodukte umwandelt. Physikalische Stabilität bezieht sich darauf, dass sich der gemessene aerodynamische Feinanteil über die Lagerzeit nicht verändert.

Ein geeigneter Pulverinhalator muss eine definierte Menge der Pulverformulierung, die abgemessene Dosis, während des Inhaliervorgangs des Patienten in ein Aerosol überführen, wobei möglichst hohe Werte für die ausgebrachte Dosis und den aerodynamischen Feinpartikelanteil erreicht werden sollten. Um das zu erreichen, ist eine wichtige Funktion des Pulverinhalators, die im Haufwerk der Pulverformulierung vorhandenen Partikelagglomerate des Arzneistoffs möglichst effizient zu deagglomerieren, da größere Teilchen beim Einatmen bereits im Mund- und Rachenraum abgeschieden werden und nur Teilchen mit aerodynamischen Partikeldurchmessern kleiner als 5 µm in die Lunge gelangen. Damit ergibt sich eine mehr oder weniger große Differenz zwischen dem Anteil der ausgebrachten Dosis bezogen auf die abgemessene Dosis und dem aerodynamischen Feinpartikelanteil, der maßgeblich durch die Effizienz der Zerstäubung beeinflusst wird.

Vor dem Hintergrund der gemachten Ausführungen muss die Teilchengröße in engen Grenzen reproduzierbar sein, damit Schwankungen der ausgebrachten Dosis und des aerodynamischen Feinanteils vermieden werden. Bei jeder Betätigung des Inhalators soll eine annähernd gleichgroße Menge Arzneistoff verabreicht werden, wobei die ausgebrachten Dosen über annähend gleiche Größenverteilungen der Arzneistoffpartikel verfügen sollen.

Aber auch unter dem Gesichtspunkt der Effizienz und einem möglichst ökonomischen Umgang mit Arzneistoffen wird angestrebt, einen möglichst großen aerodynamischen Feinpartikelanteil - wie oben definiert - zu erzeugen.

Nach dem Stand der Technik kommen grundsätzlich zwei verschiedene Systeme von Pulverinhalatoren zum Einsatz.

Die so genannten **passiven Inhalatoren** nutzen in der Regel die eingeatmete Luft des Patienten zur Zerstäubung der Pulver-Formulierung ohne zusätzliche Hilfsenergiequellen - beispielsweise in Form von Druckluft. Diese Pulverinhalatoren sind in der Art konzipiert, dass Pulver entweder in Form einer Einzeldosis (premetered dose) zum Beispiel in einer vorgefertigten Kapsel enthalten ist oder auch mehrere vordosierte Mengen in einem Mehrdosis-Behältnis, das in den Inhalator eingesetzt wird, bereitgestellt werden. Bei Gebrauch wird die Kapsel bzw. eines der Mehrdosis-Behältnisse aufgestochen, wobei die Entleerung und Zerstäubung des Pulvers mittels der eingeatmeten Luft des Patienten erfolgt.

Das Pulver kann auch als Pulvervorrat (bulk powder) im Inhalator vorliegen, wobei eine einzelne Dosis über eine Dosiervorrichtung bereitgestellt wird, bevor sie über die Atemluft des Patienten aus dem Inhalator heraus transportiert wird.

Es ist offensichtlich, dass bei den beschriebenen Pulverinhalatoren der aerodynamische Feinpartikelanteil stark von dem Atemmanöver des Patienten abhängig ist.

Vor dem Hintergrund der gemachten Ausführungen ist man dazu übergegangen so genannte aktive Pulverinhalatoren einzusetzen, welche über gespeicherte Energie, z.B. Druckgas verfügen. Durch Nutzung des Druckgases zum definierten Ausbringen und Zerstäuben der Pulver-Formulierung wird die Unabhängigkeit vom Atemmanöver des Patienten erreicht.

In der WO 01/41846 A1 wird beispielsweise u.a. ein medizinischer Zerstäuber gezeigt, bei dem pulverformiges Medikament mittels einer Druckgaspatrone als diskrete Dosis aus einer Tasche ausgebracht wird. Nach ihrer Befüllung mit Pulver wird diese Tasche mittels eines Transportmittels - beispielsweise auch atemzugsgetriggert - in Kontakt mit einer Vorrichtung zur Aerosolerzeugung gebracht, an welche die Druckgaspatrone angeschlossen ist. Bei der Aerosolerzeugung strömt das Aerosol aus der Tasche über ein Mundstück am Gerät zum Patienten.

Um Deagglomeration und effiziente Zerstäubung zu bewirken und die gewünschte Teilchengröße und Teilchengrößenverteilung zu realisieren, wurden nach dem Stand der Technik im wesentlichen zwei Wege beschritten.

Bei einigen in der Literatur beschriebenen Inhalatoren wird die Deagglomeration des Pulvers durch Aufprall der Teilchen auf so genannte Prallflächen unterstützt. Beispielsweise mit Hilfe von Druck werden die Pulverpartikeln gezielt auf diese Prallflächen gelenkt, um eine Deagglomeration der Partikel zu erzielen. Dabei stellt sich aber der Effekt ein, dass ein Teil der auf die Prallfläche auftreffenden Pulverpartikel an der Prallfläche haften bleiben und hier deponiert werden. Das hat zur Folge, dass eine möglichst genaue und reproduzierbare Dosierung nicht möglich ist, da sich die deponierten Pulverpartikel bei nachfolgenden Inhalationen wieder zufällig lösen können.

Nachteilig ist, dass bei der Verwendung von unter Druck stehenden Gasen zur Deagglomeration von Pulverformulierungen sehr hohe Aerosolgeschwindigkeiten erzeugt werden. Sehr hohe Aerosolgeschwindigkeiten wiederum führen dazu, dass der Anteil der Dosis, der die Lunge erreicht, verkleinert wird. Daher wurden für Pulverinhalatoren, die mit Druckgasen arbeiten, zusätzliche Spacer/ Separatoren vorgesehen, die die Aufgabe haben, die Geschwindigkeit der gebildeten Aerosolpartikel herabzusetzen.

Diese Spacer/ Separatoren (oder Deagglomerationskammem), in denen die Geschwindigkeit der Pulverteilchen abgebremst wird, werden vor dem Mundstück des Inhalators angeordnet und machen den Inhalator sperrig und unhandlich. Inhalatoren für den pharmazeutischen Bereich sollen aber klein und handlich sein, damit der Patient den Inhalator ständig mit sich führen kann.

Vor diesem Hintergrund ist es die Aufgabe der vorliegenden Erfindung, einen Pulverinhalator zur Dispergierung von pharmazeutischen Pulver-Formulierungen bereitzustellen, mit welchem die aus dem Stand der Technik bekannten Probleme der herkömmlichen Pulverinhalatoren eliminiert, zumindest aber gemindert werden und mit dem insbesondere ein hoher Anteil an Feststoffpartikeln kleiner als 5,8 µm erzeugt wird.

Gelöst wird diese Aufgabe durch einen Pulverinhalator der gattungsbildenen Art, der dadurch gekennzeichnet ist, dass in dem Inhalator eine Düse vorgesehen ist, durch welche das Aerosol vor Verlassen des Inhalators strömt und dass die Düse eine Mehrkanaldüse ist, deren Kanäle in einem solchen Winkel zueinander geneigt sind, dass die durch sie strömenden Aerosol-Jets stromabwärts hinter der Düse aufeinander treffen.

Bei dem erfindungsgemäßen Pulverinhalator trägt das Druckmedium das Pulver aus der Pulverbevorratung und transportiert es durch mindestens zwei Kanäle der Düse. Alternativ kann auch ein Pulverinhalator verwendet werden, bei welchem durch die Atemluft des Patienten eine Strömung erzeugt wird, um das Pulver aus der Pulverbevorratung und den Kanälen der Düse zu transportieren.

Die erfindungsgemäße Düse zeichnet sich dadurch aus, dass Pulverpartikel mit einer hohen kinetischen Energie erzeugt werden, so dass der Feinpartikelanteil entsprechend hoch ist. Die Partikel werden durch die Jet-Impaktion anschließend wieder abgebremst und die dabei frei werdende Energie wird für die Dispersion des Pulvers verwendet. Von großem Vorteil ist dabei, dass beim Abbremsen des Aerosols keine Geräteoberflächen beteiligt sind und keine bzw. nur sehr geringe Pulverrückstände zurückbleiben.

Gemäß einer besonders bevorzugten Ausführungsform ist vorgesehen, dass die Düse eine Zweikanaldüse ist, deren Kanäle in einem Winkel α zueinander geneigt sind, wobei 0°<α≤180° ist. Der geeignetste Winkel wird in Abhängigkeit unter anderem von der Zusammensetzung der Pulverformulierung und weiteren Parametern ermittelt.

Gemäß einer vorteilhaften Weiterbildung ist vorgesehen, dass die Pulverformulierung mit dem Druckmedium vermischt allen Kanälen der Düse gleichzeitig zugeführt wird. So entsteht ein vollkommen homogenes Aerosol stromabwärts der Düse.

Gemäß einer anderen Weiterbildung ist vorgesehen, dass wenigstens einem Kanal das Druckmedium und den übrigen Kanälen ein Gemisch aus Pulverformulierung und Druckmedium zugeführt wird. Mindestens ein Jetstrahl aus dem Druckmedium trifft so auf wenigstens einen Jetstrahl mit dem vordispergierten Pulver.

Zwar wird die kinetische Energie der Pulverpartikel durch die Jet-Impaktion erheblich reduziert, die nachfolgenden Überlegungen betreffen nun aber das weitere Verhalten des Aerosols stromabwärts der Düse.
Da eine zu hohe Strömungsgeschwindigkeit den Feinpartikelanteil, der während des Inhalationsvorgangs die Lunge des Patienten erreichen kann, verringert, sollte die Geschwindigkeit am Austritt des Mundstückes nicht größer als 20 m/s, vorzugsweise nicht größer als 10m/s, insbesondere nicht größer als 5 m/s sein.

Da zur Deagglomeration des Pulvers beim Durchgang durch die Düse hohe Strömungsgeschwindigkeiten erzeugt werden müssen, ist es generell notwendig, die Aerosolgeschwindigkeit auf der Wegstrecke zwischen Düse und Mundstück zu reduzieren. Dies lässt sich auf verschiedene Art und Weise bewerkstelligen, so dass auf die Verwendung des erfindungsgemäßen Pulverinhalators zusammen mit einem Spacer verzichtet werden kann. Im Einzelnen ergibt sich bevorzugt folgendes:

Das an den Düsenauslässen austretende Aerosol kann auf verschiedene Weise mit der Atemluft des Patienten auf der Wegstrecke zwischen Düse und Mundstück vermischt werden.

Die Erzeugung einer wirbelförmigen Strömung der Einatemluft des Patienten ist eine dafür geeignete Maßnahme. Die durch die Düsenkanäle tretende Aerosolwolke wird in den Wirbel der Einatemluft injiziert, wodurch sich die Vorwärtskomponente der Geschwindigkeit der Aerosolwolke reduziert.
Eine Auslegung der Düse in der Art, dass die Einatemluft und die aus den Düsenkanälen austretende Aerosolströmung entgegengerichtet sind, wodurch ebenfalls eine Verzögerung erzielt werden kann, stellt eine andere Maßnahme zur Verringerung der Strömungsgeschwindigkeit der Aerosolwolke dar.

In einer alternativen Ausführungsform befindet sich die Eintrittsöffnung der Einatemluft windschief oder senkrecht zur Strömungsrichtung der Düse.

Um die Geschwindigkeit der aus den Düsenkanälen austretenden Aerosolwolke im Inhalator zu reduzieren, kann es sich als vorteilhaft erweisen, diese Aerosolwolke mit einem entgegenkommenden Luftrom zu vermischen. Vorteilhaft ist es dabei, wenn dieser entgegenkommende Luftstrom durch den Einatmungsvorgang des Benutzers erzeugt wird. Üblicherweise wird bei einem Pulverinhalator dafür gesorgt, dass der Patient beim Einatmen der Pulverwolke auch Luft mit einatmet, um einen komplikationslosen Einatmungsvorgang gewährleisten zu können. Dafür können z.B. im Mundstück Luftschlitze ausgebildet sein, durch die der Patient automatisch beim Einatmen der Partikelwolke auch Luft mit einatmet. Im Rahmen des erfindungsgemäßen Pulverinhalators können diese Lufteinlassöffnungen so ausgebildet sein, dass die einströmende Luft der Aerosolwolke entgegenläuft entweder als rein entgegenlaufender oder auf die Aerosolwolke aufprallender Luftstrom, der so deren Geschwindigkeit abbremst. Konstruktiv kann diese Aufgabe dadurch gelöst werden, dass z.B. das Mundstück senkrecht zu dem Weg steht, den die Aerosolwolke beim Austreten aus den Düsenöffnungen nimmt. Die Lufteinlassschlitze sind dann ebenfalls senkrecht zum Mundstück und in einer Linie mit dem Austrittsweg der Aerosolwolke aus der Düse, aber der Düse genau entgegengesetzt ausgebildet. Eine solche Konstruktion hätte vereinfacht ausgedrückt die Form eines T-Stückes, wobei die Düse für die Aerosolwolke und die Lufteinlässe für die Gegenluft jeweils die Endpunkte des T-Balkens bestimmen und das Mundstück dem Fußpunkt des T entspricht.

In einer alternativen Ausführungsform münden die Düsenkanäle und die Lufteinlasslöcher in einer Wirbelkammer, werden darin miteinander verwirbelt und treten dann durch ein Mundstück aus.

Eine solche Wirbelkammer kann im einfachsten Fall ein hohler Raum sein, in den an einer Stelle die Düse einmündet: Die Lufteinlässe können dann genau gegenüber der Düse oder bevorzugt senkrecht dazu oder zumindest windschief versetzt in den Raum münden. Der Raum hat dann einen Ausgang zum Mundstück.

Im einfachsten Fall besteht der erfindungsgemäße Pulverinhalator aus einem Gehäuse mit einem Mundstück. Dem Mundstück vorgelagert, im Inneren des Gehäuses befindet sich die oben beschriebene Düse. Weiter im Inneren befindet sich ein Raum zur Aufnahme der Pulverformulierung, die zerstäubt werden soll.

Bevorzugt weist der Pulverinhalator ein Drucksystem auf, welches ein unter Druck stehendes Gas auf die zu dispergierende Menge der Pulverformulierung leitet, so dass diese dispergiert wird und das entstehende Aerosol durch das oben beschriebene Düsensystem unter Aufbrechen der gröberen Teilchen in das Mundstück geleitet wird. Der Pulverinhalator weist dabei in seinem Inneren Kanäle auf, die den Weg des unter Druck abgegebenen Gases durch den Inhalator bis in das Mundstück vorgeben. In dem Raum zur Aufnahme der zur Dispergierung vorgesehenen Pulverformulierung kann das Pulver lose liegen oder es befindet sich in einem Container, z.B. in einer Kapsel oder einem Blister, der vor Durchleiten des Druckgases so geöffnet wird, dass das Druckgas das Pulver weitgehend ohne Rückstände mitreißen kann. Im Fall eines Einweginhalators weist dieser keine weitere Bevorratung für weitere Dosierungen der Pulverformulierungen auf. Im Fall eines Geräts zur mehrmaligen Benutzung kann der Inhalator ein oder mehrere Reservoir(e) für die Pulverformulierung aufweisen. So kann das Reservoir lediglich ein Raum sein, der das lose Pulvergemisch beinhaltet und von dort werden abgemessene Mengen in den Zerstäubungsraum überführt. Alternativ kann das Reservoir eine Ansammlung von Kapseln sein, die mit der Pulverformulierung gefüllt sind und mechanisch oder manuell in die Zerstäubungskammer eingebracht werden. Schließlich kann es sich bei dem Reservoir auch um einen Blister mit vielen Taschen für die Pulverformulierung handeln, wobei dann immer eine dieser Taschen in den Zerstäubungssraum eingebracht wird. Derartige Reservoirsysteme und die Überführung der Pulverformulierung aus dem Reservoir in den Vernebelungsraum sind aus dem Stand der Technik bekannt, weshalb an dieser Stelle nicht näher darauf eingegangen werden soll.

Als Druckmediumsystem kann der erfindungsgemäße Pulverinhalator eine Druckluftpatrone aufweisen, eine mit einem anderen Gas als Luft gefüllte Patrone, z.B. Stickstoff, Kohlendioxid, ein Edelgas, wie Helium oder Argon, oder sogar ein Fluorkohlenwasserstoff, ein Alkan und dergleichen. Vorteilhaft sind Ausführungsformen des Pulverinhalators, bei denen das Druckmediumsystem ein System ist, welches Luft aus der Umgebung ansaugt und dann die Luft gezielt unter Kompression und Druck in Richtung der zu vernebelnden Formulierung abgibt. Zum einen ist Luft als Trägermedium für die Pulverpartikel das wohl unbedenklichste Medium für den Patienten. Zum anderen ist es leicht verfügbar. Die bevorzugte Ausführungsform des Pulverinhalators entnimmt der Umgebung die benötigte Menge an Luft, komprimiert sie und nutzt sie dann als Trägermedium für die Pulver-Formulierung. Ein Auswechseln des Druckmediumsystem, wie dies der Fall bei einem in einer Patrone gespeicherten Druckmedium der Fall wäre, entfällt.

Vorteilhaft können aber auch Ausführungsformen des Pulverinhalators sein, bei denen das Druckmediumsystem eine Patrone umfasst, welche ein unter Druck stehendes Druckmedium bevorratet. Im Gegensatz zu dem zuvor beschriebenen Pulverinhalator, ist diese Ausführungsform weniger komplex in ihrem Aufbau und daher kostengünstiger und in ihren Abmessungen kleiner.

In jedem Fall ist das Druckmediumsystem derart, dass der Verwender des Inhalators gezielt einen Ausstoß des Druckmediums erzeugen kann.

Vorteilhaft sind Ausführungsformen des Pulverinhalators, die dadurch gekennzeichnet sind, dass die Vorrichtung zur Bevorratung der Pulver-Formulierung zwischen dem Druckmediumsystem und Düse angeordnet ist in der Art, dass das Druckmedium die Vorrichtung passieren muss, wobei vorzugsweise die Vorrichtung zur Bevorratung der Pulver-Formulierung eine mit Pulver gefüllte Kapsel aufweist. Bevorzugte Ausführungsformen des Pulverinhalators sind die, bei denen die Kapsel als Verbrauchsmaterial austauschbar ist. Hierbei wird ein Mechanismus am Pulverinhalator vorgesehen, mit dem ein Austausch der Kapsel erfolgen kann.

Das Druckmedium durchströmt bei dieser Ausführungsform die Vorrichtung zur Bevorratung der Pulver-Formulierung und verteilt bzw. nimmt das Pulver in sich auf, so dass nach Durchströmen der Bevorratungskammer das gewünschte Primär-Aerosol vorliegt.

Alternativ kann auch eine Ausführungsform ohne Druckmedium verwendet werden. In diesem Fall wird die Durchströmung durch die Atemluft des Patienten beim Einatmen erzeugt, damit das gewünschte Primär-Aerosol vorliegt.

Vorteilhaft sind Ausführungsformen des Pulverinhalators, bei denen die Vorrichtung zur Bevorratung der Pulver-Formulierung ein Mehrdosis-Blister-Behältnis umfasst. Dabei können solche Mehrdosis-Blister-Behältnisse linear als Blisterband, flächig als Blister-Scheiben oder Blister-Ringe oder dreidimensional als zylindrische oder vielflächige Körper ausgeführt sein. Derartige Mehrdosissysteme können 2 bis 90 Dosen enthalten, bevorzugt 5 bis 60 Dosen, insbesondere 7 bis 30 Dosen, wobei jede Dosis in einer separaten Tasche gelagert ist, die bei Gebrauch mittels einer geeigneten Vorrichtung geöffnet wird.

Wie bereits ausgeführt, werden Pulverinhalatoren bevorzugt, bei denen Luft als Druckmedium vorgesehen wird.

Sofern das Druckmediumsystem nicht prinzipbedingt per Hand betätigt wird, sondern ein zu betätigendes Stellglied - beispielsweise in Form eines Aktuator-Ventils - aufweist, durch dessen Öffnen oder Schließen das Druckmedium freigegeben wird, sind Ausführungsformen des Pulverinhalators vorteilhaft, die im Mundstück ein Durchflusssensor vorsehen, der den Einatemfluss des Patienten misst und ab einem bestimmten Wert ein Eingangssignal für das Druckmediumsystem bzw. dessen Stellglied generiert.

Der Durchflusssensor misst den Luftstrom beim Einatmen des Patienten und generiert ein Eingangssignal, mit welchem das Stellglied beaufschlagt wird. Es öffnet und lässt das Druckmedium ausströmen, wenn die Durchflussrate in einem für die Inhalation geeigneten Bereich liegt, wobei das Stellglied das Druckmediumsystem nicht freigibt, falls diese Durchflussrate außerhalb des bevorzugten Bereiches liegt. Dieses automatische Öffnen und Schließen erspart das Vorsehen einer zusätzlichen Betätigungsvorrichtung und sorgt für mehr Bedienungsfreundlichkeit und eine leichtere Handhabung des Inhalators.

Der erfindungsgemäße Pulverinhaltor weist folgende Vorteile gegenüber dem Stand der Technik auf:
- aus einem in einem geeigneten Behältnis vordosierten Pulverhaufwerk wird ein Aerosol erzeugt, wobei die in Form eines unter Druck stehenden Gases vorhandene Energie zur Überwindung der Agglomerationskräfte der mikronisierten Pulverteilchen untereinander verwendet wird, so dass ein Aerosol mit vergleichsweise überdurchschnittlich hohen Anteil von Feststoffpartikeln kleiner als 5,8 µm erzeugt wird.
- das hier beschriebene System muss nicht auf fluorierten Treibgasen oder Fluor-ChlorKohlenwasserstoffen zurückgreifen.
- die Erzeugung des Aerosols und die erreichte Partikelgrößenverteilung ist unabhängig vom Atemmanöver des Patienten.
- die Pulverrückstände im Gerät sind konstruktionsbedingt minimal, da die Deagglomeration des Pulvers durch das Aufeinanderprallen (Jet-Impaktion) mehrerer

Jet-Strahlen und nicht durch Impaktion an Prallflächen erreicht wird.
- der beschriebene Inhalator ist klein und kann durch den Patienten leicht mit sich geführt werden.
- Auf die Verwendung des Gerätes mit einem "Spacer" - wie für andere "aktive" Pulversysteme (Patent WO 99/6249) - kann verzichtet werden, da im Gerät durch entsprechende Vorkehrungen eine langsam fortbewegte Aerosolwolke erzeugt wird.

Im Folgenden wird die Erfindung anhand mehrerer Ausführungsformen gemäß den beiden Zeichnungsfiguren näher erläutert. Hierbei zeigt:
- Fig. 1: eine schematische Darstellung einer ersten Ausführungsform eines Pulverinhalators in der Seitenansicht, geschnitten,
- Fig. 2a: eine schematische Darstellung der Mehrkanaldüse eines Pulverinhalators in der Seitenansicht, geschnitten,
- Fig. 2b: die Düse gemäß Fig. 2a in einer anderen Betriebsart,
- Fig. 3a: eine schematische Darstellung einer ersten Ausführungsform eines Mehrdosis-Blister-Behältnisses zur Bevorratung der Pulver-Formulierung in der Seitenansicht,
- Fig. 3b: eine schematische Darstellung einer zweiten Ausführungsform eines Mehrdosis-Blister-Behältnisses zur Bevorratung der Pulver-Formulierung in der Seitenansicht,
- Fig. 3c: eine schematische Darstellung einer dritten Ausführungsform eines Mehrdosis-Blister-Behältnisses zur Bevorratung der Pulver-Formulierung in der Seitenansicht,
- Fig. 3d: eine schematische Darstellung einer vierten Ausführungsform eines Mehrdosis-Blister-Behältnisses zur Bevorratung der Pulver-Formulierung in der perspektivischen Darstellung,
- Fig. 3e: eine schematische Darstellung einer fünften Ausführungsform eines Mehrdosis-Blister-Behältnisses zur Bevorratung der Pulver-Formulierung in der perspektivischen Darstellung,
- Fig. 4: eine schematische Darstellung einer ersten Ausführungsform einer Vorrichtung zur Verzögerung der Aerosolströmung in der Seitenansicht, geschnitten, und
- Fig. 5: eine schematische Darstellung einer zweiten Ausführungsform einer Vorrichtung zur Verzögerung der Aerosolströmung in der Seitenansicht, geschnitten.

Nachfolgend sind gleiche Teile mit denselben Bezugszeichen versehen.

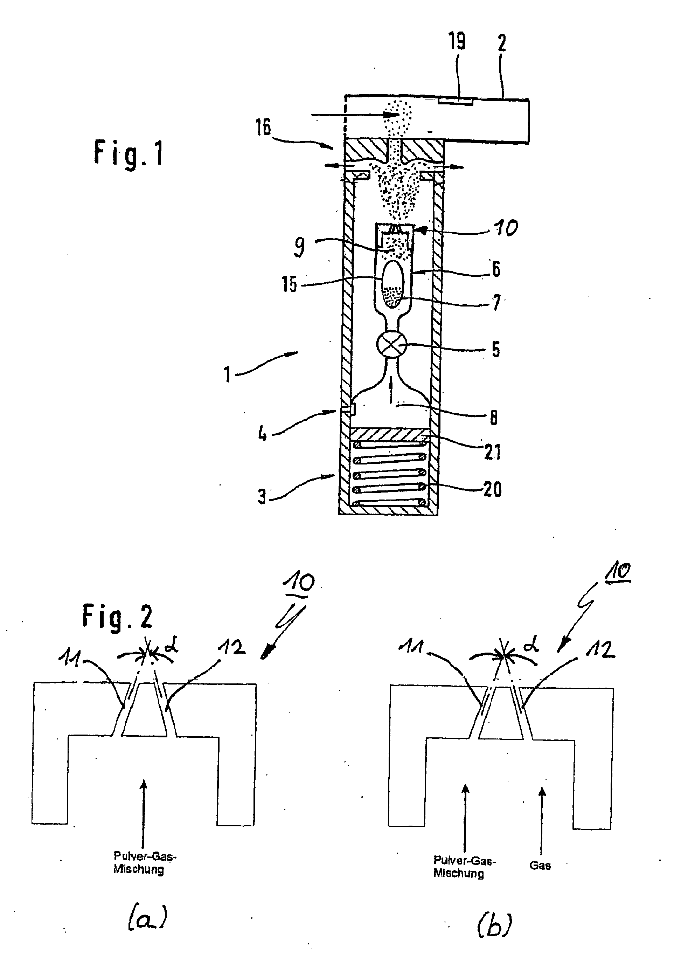

Fig. 1 zeigt in einer schematischen Darstellung eine erste Ausführungsform eines Pulverinhalators 1 zur Dispergierung von pharmazeutischen Arzneistoff-Formulierungen in der Seitenansicht und geschnitten.

Der Pulverinhalator 1 verfügt an seinem oberen Ende über ein Mundstück 2. Er weist eine Hilfsenergiequelle in Form eines Druckmediumsystems 3 auf, wobei das Druckmediumsystems 3 mit einer Pumpe ausgestattet ist, die mit der Umgebung über ein Ventil 4 in Verbindung steht und die Umgebungsluft als Druckmedium 8 verwendet. Die Luft dient als Trägermedium für die Pulverpartikel 7 und wird von einem über eine Feder 20 kraftbeaufschlagten Kolben 21 während der Expansionsphase über das Ventil 4 angesaugt und beim Hochfahren des Kolbens komprimiert. Ein Auswechseln des Druckmediumsystem, wie dies der Fall bei einem in einer Patrone gespeicherten Druckmedium der Fall wäre, entfällt.
Das Druckmedium 8 verlässt das Druckmediumsystem 3 über ein Stellglied bzw. ein Aktuator-Ventil 5. Bei der in Fig. 1 dargestellten Ausführungsform ist ein Durchflusssensor 19 im Mundstück 2 vorgesehen. Der Durchflusssensor 19 misst den Luftstrom beim Einatmen des Patienten - die Durchflussrate - und generiert ein Eingangssignal, mit welchem das Aktuator-Ventil 5 beaufschlagt wird. Es öffnet und lässt das Druckmedium 8 ausströmen, wenn die Durchflussrate in einem für die Inhalation geeigneten Bereich liegt, wobei das Stellglied 5 das Druckmediumsystem 3 schließt, falls diese Durchflussrate außerhalb dieses bevorzugten Bereiches liegt. Dieses automatische Öffnen und Schließen sorgt für mehr Bedienungsfreundlichkeit und optimiert die Bedingungen während der Inhalation.

Nach Austritt aus dem Druckmediumsystem 3 durchströmt das Druckmedium 8 eine Vorrichtung zur Bevorratung 6 der Pulver-Formulierung 7. Die Vorrichtung zur Bevorratung 6 der Pulver-Formulierung 7 verfügt über eine mit Pulver 7 gefüllte Kapsel 15. Der Mechanismus, mit dem ein Austausch der Kapsel erfolgen kann, ist nicht dargestellt.

Das Druckmedium 8 durchströmt bei dieser Ausführungsform die Vorrichtung zur Bevorratung 6 der Pulver-Formulierung 7 und nimmt einen Teil des Pulvers 7 in sich auf, so dass, nach Durchströmen der Bevorratungskammer das gewünschte Primär-Aerosol 9 in der Art vorliegt, dass die Pulverpartikel 7 in dem gasförmigen Druckmedium 8 dispergiert sind.

Fig. 2a zeigt nun schematisch die Mehrkanaldüse 10 in Ausbildung einer Zweikanaldüse mit zwei Kanälen 11 und 12. Die Kanäle 11 und 12 sind zueinander in einem Winkel α geneigt. Hierbei kann der Winkel α zwischen 0° und 180° liegen. Die Neigung führt dazu, dass ein Aerosol-Jet aus dem Kanal 11 mit einem Aerosol-Jet aus dem Kanal 12 impaktiert und die Pulverpartikel deagglomeriert werden. In der Ausbildung nach Fig. 2a werden beide Kanäle 11 und 12 einheitlich mit einer Pulver-Gas-Mischung beschickt.

Fig. 2b zeigt die Düse 10 gemäß Fig. 2a in einer anderen Betriebsweise. Hier wird einem Kanal 12 Druckmedium 8 als reines Gas zugeführt, wohingegen dem Kanal 11 wiederum eine Pulver-GasMischung zugeführt wird.
Die Aerosol-Jets können - wie ausgeführt - in jedem beliebigen Winkel von 0° bis 180°, in jedem beliebigen Abstand von der Oberfläche der Düse 10 und mit jeder beliebigen Geschwindigkeit aufeinander prallen. Das Verhältnis der Parameter entscheidet über den Grad der Dispergierung und über die Geschwindigkeit der entstehenden Sekundär-Aerosolwolke.

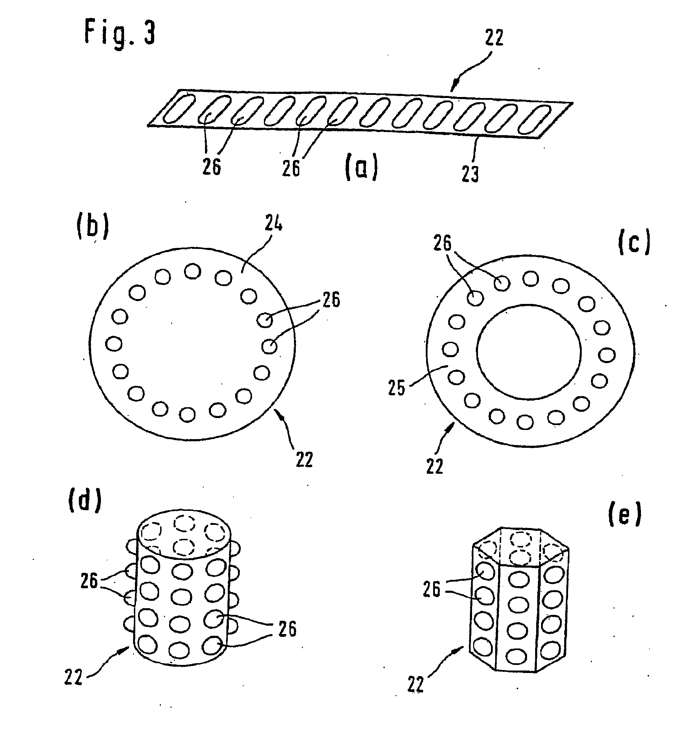

Fig. 3a zeigt eine schematische Darstellung einer ersten Ausführungsform eines Mehrdosis-Blister-Behältnisses 22 zur Bevorratung der Pulver-Formulierung in der Seitenansicht. Das Mehrdosis-Blister-Behältnisse 22 ist linear als Blisterband 23 ausgebildet und verfügt über mehrere in einer Reihe angeordnete Kapsel 26, von denen jede Kapsel 26 eine Einzeldosis beinhaltet, wobei die jeweilige Kapsel 26 bei Gebrauch mittels einer geeigneten Vorrichtung (nicht dargestellt) geöffnet wird.

Fig. 3b zeigt eine schematische Darstellung einer zweiten Ausführungsform eines Mehrdosis-Blister-Behältnisses 22 zur Bevorratung der Pulver-Formulierung in der Seitenansicht. Das Mehrdosis-Blister-Behältnis 22 ist flächig als Blister-Scheibe 24 ausgebildet und verfügt über eine Vielzahl von in einem Kreis angeordnete Kapseln 26.

Fig. 3c zeigt eine schematische Darstellung einer dritten Ausführungsform eines Mehrdosis-Blister-Behältnisses 22 zur Bevorratung der Pulver-Formulierung in der Seitenansicht. Das Mehrdosis-Blister-Behältnis 22 ist flächig als Blister-Ring 25 ausgebildet und verfügt über eine Vielzahl von in einem Kreis angeordnete Kapseln 26.

Fig. 3d zeigt eine schematische Darstellung einer vierten Ausführungsform eines Mehrdosis-Blister-Behältnisses 22 zur Bevorratung der Pulver-Formulierung in der perspektivischen Darstellung. Das Mehrdosis-Blister-Behältnis 22 ist dreidimensional als zylindrischer Körper ausgeführt. Derartige Mehrdosissysteme können 2 bis 90 Dosen enthalten, bevorzugt 5 bis 60 Dosen, insbesondere 7 bis 30 Dosen, wobei jede Dosis in einer separaten Kapsel 26 gelagert ist, die bei Gebrauch mittels einer geeigneten Vorrichtung geöffnet wird.

Fig. 3e zeigt eine schematische Darstellung einer fünften Ausführungsform eines Mehrdosis-Blister-Behältnisses 22 zur Bevorratung der Pulver-Formulierung in der perspektivischen Darstellung. Das Mehrdosis-Blister-Behältnis 22 ist - wie in Fig. 3d - dreidimensional als vielflächiger Körper ausgeführt.

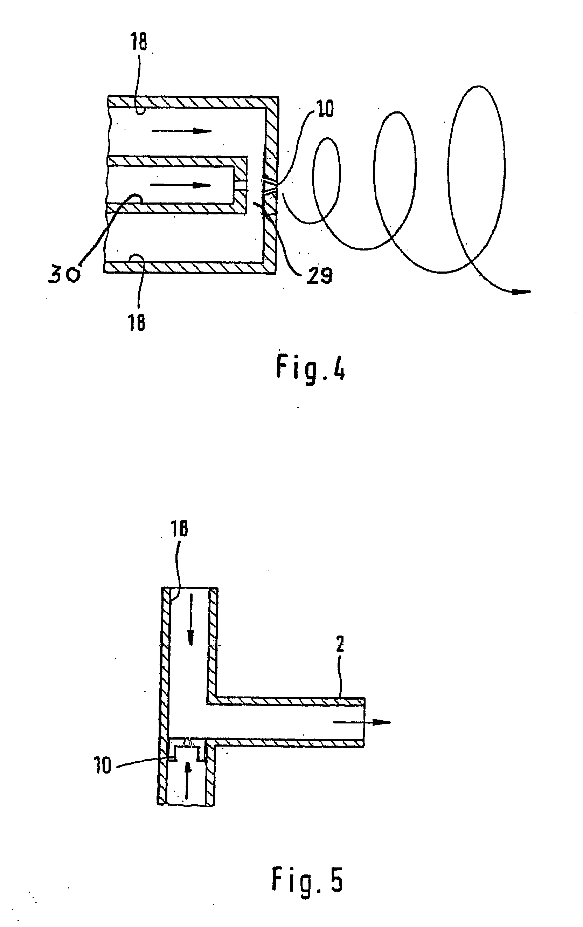

Fig. 4 zeigt eine schematische Darstellung einer ersten Ausführungsform einer Vorrichtung zur Verzögerung der Aerosolströmung in der Seitenansicht, geschnitten. Beide Strömungen, sowohl die Einatmluftströmung im Einlasskanal 18 als auch die Aerosolströmung im Kanal 30, verlaufen zunächst parallel in zwei zueinander koaxialen Rohren, wobei die Einatemluft in der Wirbelkammer 29 in eine wirbelförmige Strömung transformiert wird. Dort findet auch die Vermischung des Sekundär-Aerosols mit der Einatemluft statt. Die in diese wirbelförmige Strömung injizierte Sekundär-Aerosolströmung weist eine verminderte Strömungsgeschwindigkeit auf.

Fig. 5 zeigt eine schematische Darstellung einer zweiten Ausführungsform einer Vorrichtung zur Verzögerung der Aerosolströmung in der Seitenansicht, geschnitten. Die Einatemluftströmung und die Sekundär-Aerosolströmung werden aufeinander zugeleitet. Bei ihrem Zusammentreffen und infolge der Strömungsumlenkung wird die Vorwärtsgeschwindigkeit der Aerosolströmung vermindert.

### Bezugszeichenliste

- 1: Pulverinhalator
- 2: Mundstück
- 3: Druckmediumsystem
- 4: Ventil
- 5: Aktuator-Ventil
- 6: Vorrichtung zur Bevorratung
- 7: Pulver-Formulierung
- 8: Aerosol
- 9: Düse
- 10: Düsenkanal
- 11: Düsenkanal
- 15: Kapsel
- 18: Einlasskanal
- 19: Durchflusssensor
- 20: Feder
- 21: Kolben
- 22: Mehrdosis-Blister-Behältnis
- 23: Blisterband
- 24: Blister-Scheibe
- 25: Blister-Ringe
- 26: Kapsel
- 27: Blisterkörper
- 28: Lochblende
- 29: Wirbelkammer
- 30: Kanal

## Patentansprüche

1. Pulverinhalator (1) mit Mundstück (2) zur Dispergierung von pharmazeutischen Arzneistoff-Formulierungen, welcher über eine Hilfsenergiequelle in Form eines Druckmediumsystems (3) verfügt, mit einer Vorrichtung zur Bevorratung (6) einer Pulver-Formulierung (7), wobei bei Aktivierung des Druckmediumsystems ein von dem Druckmediumsystem (3) freigesetztes, gasförmiges Druckmedium (8) mit der Pulver-Formulierung (7) ein Aerosol (9) in der Art ausbildet, dass die Pulverpartikel in dem gasförmigen Druckmedium (8) dispergiert vorliegen, **dadurch gekennzeichnet,**
**dass** das Druckmediumsystem (3) eine mit der Umgebung in Verbindung stehende Pumpe mit einem über eine Feder (20) kraftbeaufschlagten Kolben (21) aufweist und die Umgebungsluft als Druckmedium (8) verwendet und
**dass** in dem Inhalator (1) eine Düse (10) vorgesehen ist, durch welche das Aerosol (9) vor Verlassen des Inhalators (1) strömt und dass die Düse (10) eine Mehrkanaldüse ist, deren Kanäle in einem solchen Winkel zueinander geneigt sind, dass die durch sie strömenden Aerosol-Jets stromabwärts hinter der Düse (10) aufeinander treffen und es dabei zu einer Jet-Impaktion kommt.

2. Pulverinhalator (1) nach Anspruch 1, **dadurch gekennzeichnet, dass** die Pulverformulierung (7) mit dem Druckmedium (8) vermischt allen Kanälen zugeführt wird.

3. Pulverinhalator (1) nach einem der vorherigen Ansprüche, **dadurch gekennzeichnet, dass** die Vorrichtung zur Bevorratung (6) der Pulver-Formulierung (7) zwischen dem Druckmediumsystem (3) und Düse (10) angeordnet ist in der Art, dass das Druckmedium (8) die Vorrichtung (6) passieren muss.

4. Pulverinhalator (1) nach einem der vorherigen Ansprüche, **dadurch gekennzeichnet, dass** die Vorrichtung zur Bevorratung (6) der Pulver-Formulierung (7) eine mit Pulver (7) gefüllte Kapsel (15) aufweist.

5. Pulverinhalator (1) nach Anspruch 4, **dadurch gekennzeichnet, dass** die Kapsel (15) als Verbrauchsmaterial austauschbar ist.

6. Pulverinhalator (1) nach einem der Ansprüche 1 bis 3, **dadurch gekennzeichnet, dass** die Vorrichtung zur Bevorratung (6) der Pulver-Formulierung (7) ein Mehrdosis-Blister-Behältnis umfasst.

7. Pulverinhalator (1) nach einem der Ansprüche 1 bis 6, **dadurch gekennzeichnet, dass** im Mundstück (2) ein Durchflusssensor (19) vorgesehen ist, der ein Eingangssignal für das Druckmediumsystem (3) generiert.

8. Pulverinhalator (1) nach einem der vorhergehenden Ansprüche, **dadurch gekennzeichnet, dass** zwischen der Düse (10) und dem Austritt des Mundstückes (2) eine wirbelförmige Strömung der durch ein Einlasskanal angesogenen Einatemluft erzeugt wird.

9. Pulverinhalator (7) nach einem der vorhergehenden Ansprüche, **dadurch gekennzeichnet, dass** die Düse (10) und ein Einlasskanal (18) für die Einatemluft so angeordnet sind, dass die aus der Düse (10) austretende Aerosolströmung und die Einatemluft entgegen gerichtet sind.

10. Pulverinhalator (1) nach einem der Ansprüche 1 bis 9, **dadurch gekennzeichnet, dass** die Düse (10) und ein Einlasskanal für die Einatemluft so angeordnet sind, dass die aus der Düse (10) austretende Aerosolströmung und die Einatemluft in einem Winkel aufeinander prallen.

11. Pulverinhalator (1) nach einem der Ansprüche 1 bis 10, **dadurch gekennzeichnet, dass** der die Aerosolströmung führende Kanal (30) und die Einlasskanäle (18) für die Einatemluft in einer Wirbelkammer (29) münden, von der aus die Aerosolwolke zur Düse (10) geleitet wird.

## Claims

1. Powder inhaler (1) with mouthpiece (2) for dispersing pharmaceutical medicament formulations, which comprises an auxiliary energy source in the form of a pressure medium system (3), having a device for supplying (6) a powder formulation (7), while on activation of the pressure medium system a gaseous pressure medium (8) released by the pressure medium system (3) forms an aerosol (9) with the powder formulation (7) such that the powder particles are present in the gaseous pressure medium (8) in dispersed form,
**characterised in that** the pressure medium system (3) comprises a pump connected to atmosphere with a piston (21) acted upon by the force of a spring (20) and uses atmospheric air as the pressure medium (8) and
**in that** a nozzle (10) is provided in the inhaler (1) through which the aerosol (9) flows before leaving the inhaler (1) and **in that** the nozzle (10) is a multi-channel nozzle the channels of which are inclined to one another at an angle such that the aerosol jets flowing through them meet one another downstream behind the nozzle (10) and jet impaction occurs as a result.

2. Powder inhaler (1) according to claim 1, **characterised in that** the powder formulation (7) is supplied to all the channels after being mixed with the pressure medium (8).

3. Powder inhaler (1) according to one of the preceding claims, **characterised in that** the device for supplying (6) the powder formulation (7) is arranged between the pressure medium system (3) and nozzle (10) such that the pressure medium (8) has to pass the device (6).

4. Powder inhaler (1) according to one of the preceding claims, **characterised in that** the device for supplying (6) the powder formulation (7) comprises a capsule (15) filled with powder (7).

5. Powder inhaler (1) according to claim 4, **characterised in that** the capsule (15) is replaceable as a consumable material.

6. Powder inhaler (1) according to one of claims 1 to 3, **characterised in that** the device for supplying (6) the powder formulation (7) comprises a multi-dose blister container.

7. Powder inhaler (1) according to one of claims 1 to 6, **characterised in that** a throughflow sensor (19) is provided in the mouthpiece (2), which generates an input signal for the pressure medium system (3).

8. Powder inhaler (1) according to one of the preceding claims, **characterised in that** between the nozzle (10) and the exit of the mouthpiece (2) a turbulent flow is produced in the inspired air sucked in through an inlet channel.

9. Powder inhaler (1) according to one of the preceding claims, **characterised in that** the nozzle (10) and an inlet channel (18) for the inspired air are arranged so that the aerosol flow emerging from the nozzle (10) and the inspired air are directed counter to one another.

10. Powder inhaler (1) according to one of claims 1 to 9, **characterised in that** the nozzle (10) and an inlet channel for the inspired air are arranged so that the aerosol leaving the nozzle (10) and the inspired air strike one another at an angle.

11. Powder inhaler (1) according to one of claims 1 to 10, **characterised in that** the channel (30) guiding the aerosol flow and the inlet channels (18) for the inspired air open into a turbulence chamber (29) from which the aerosol cloud is conveyed to the nozzle (10).

## Revendications

1. Inhalateur de poudre (1) comprenant un embout (2) servant à la dispersion de formulations pharmaceutiques d'une substance médicamenteuse, lequel inhalateur de poudre dispose d'une source d'énergie auxiliaire sous la forme d'un système à milieu de pression (3), comprenant un dispositif servant à stocker (6) une formulation sous forme de poudre (7), un milieu de pression (8) sous forme gazeuse libéré par le système à milieu de pression (3) comprenant la formulation sous forme de poudre (7) formant, lors de l'activation du système à milieu de pression, un aérosol (9) de telle manière que les particules de poudre sont présentes sous la forme dispersée dans le milieu de pression (8) sous forme gazeuse,
**caractérisé en ce**
**que** le système à milieu de pression (3) présente une pompe reliée à l'environnement, dotée d'un piston (21) soumis à l'action d'une force exercée par l'intermédiaire d'un ressort (20), et en ce qu'on utilise comme milieu de pression (8) l'air ambiant, et
en ce qu'est prévue dans l'inhalateur (1) une buse (10), par laquelle l'aérosol (9) s'écoule avant de quitter l'inhalateur (1), et en ce que la buse (10) est une buse à plusieurs canaux, dont les canaux sont inclinés les uns par rapport aux autres selon un angle tel que les jets d'aérosol circulant à travers lesdits canaux se rencontrent en aval à l'arrière de la buse (10), donnant lieu ce faisant à une collision de jet.

2. Inhalateur de poudre (1) selon la revendication 1, **caractérisé en ce que** la formulation sous forme de poudre (7) est amenée aux canaux en étant mélangée au milieu de pression (8).

3. Inhalateur de poudre (1) selon l'une quelconque des revendications précédentes, **caractérisé en ce que** le dispositif servant à stocker (6) la formulation sous forme de poudre (7) est disposé entre le système à milieu de pression (3) et la buse (10) de telle manière que le milieu de pression (8) doit passer par le dispositif (6).

4. Inhalateur de poudre (1) selon l'une quelconque des revendications précédentes, **caractérisé en ce que** le dispositif de stockage (6) de la formulation sous forme de poudre (7) présente une capsule (15) remplie de poudre (7).

5. Inhalateur de poudre (1) selon la revendication 4, **caractérisé en ce que** la capsule (15) en tant que matériau consommable peut être changée.

6. Inhalateur de poudre (1) selon l'une quelconque des revendications 1 à 3, **caractérisé en ce que** le dispositif servant à stocker (6) la formulation sous forme de poudre (7) comprend un contenant blister à plusieurs doses.

7. Inhalateur de poudre (1) selon l'une quelconque des revendications 1 à 6, **caractérisé en ce qu'**un capteur de débit (19) est prévu dans l'embout (2), lequel capteur de débit génère un signal d'entrée pour le système à milieu de pression (3).

8. Inhalateur de poudre (1) selon l'une quelconque des revendications précédentes, **caractérisé en ce qu'**un écoulement sous forme de spirale de l'air inspiré par un canal d'admission est produit entre la buse (10) et la sortie de l'embout (2) .

9. Inhalateur de poudre (1) selon l'une quelconque des revendications précédentes, **caractérisé en ce que** la buse (10) et le canal d'admission (18) pour l'air inspiré sont disposés de telle manière que l'écoulement d'aérosol sortant de la buse (10) et l'air inspiré sont orientés dans des sens opposés.

10. Inhalateur de poudre (1) selon l'une quelconque des revendications 1 à 9, **caractérisé en ce que** la buse (10) et. un canal d'admission pour l'air inspiré sont disposés de telle manière que l'écoulement d'aérosol sortant de la buse (10) et l'air inspiré s'entrechoquent selon un angle.

11. Inhalateur de poudre (1) selon l'une quelconque des revendications 1 à 10, **caractérisé en ce que** le canal (30) guidant l'écoulement d'aérosol et les canaux d'admission (18) pour l'air inspiré débouchent dans une chambre de tourbillonnement (29), depuis laquelle le nuage d'aérosol est conduit en direction de la buse (10) .
